# EUROPÄISCHE PATENTSCHRIFT

(11) **EP 2 111 851 B1**
(45) Veröffentlichungstag und Bekanntmachung des Hinweises auf die Patenterteilung: **15.06.2011**
(21) Anmeldenummer: 08154960.2
(22) Anmeldetag: 22.04.2008
(51) Int. Cl.: A61K 8/37, A61K 8/34, A61Q 19/00

(54) **Emulgatorgemisch**
Emulgator mixture
Mélange d'émulsifiants

(43) Veröffentlichungstag der Anmeldung: 28.10.2009
(73) Patentinhaber: Azelis Deutschland Kosmetik GmbH, 47445 Moers (DE)
(72) Erfinder: Seidel, Holger, 47055, Duisburg (DE)
(74) Vertreter: Féaux de Lacroix, Stefan

(56) Entgegenhaltungen:
- US-A- 5 744 062
- US-A1- 2006 194 057
- US-B1- 6 710 022

## Beschreibung

Die Erfindung betrifft ein Emulgatorgemisch, dieses Gemisch enthaltende Emulsionen und deren Verwendung als Emulsionskonzentrat zur Herstellung kosmetischer oder pharmazeutischer Zusammensetzungen.

Kosmetische und dermatologische Zubereitungen, die partiell neutralisierte Ester von Monoglyceriden und/oder Diglyceriden gesättigter Fettsäuren mit Citronensäure enthalten, sind bekannt. Die EP-A-1 049 452 betrifft dünnflüssige, kosmetische und dermatologische Zubereitungen, deren Viskosität bei 25°C kleiner ist als 2500 mPas, die einen oder mehrere partiell neutralisierte Ester von Monoglyceriden und/oder Diglyceriden gesättigter Fettsäuren mit Citronensäure, einen oder mehrere Fettalkohole mit 12 bis 40 Kohlenstoffatomen, eine Wasserphase und 2 bis 7,5 Gew.-% einer Lipidphase enthalten, wobei das Gewichtsverhältnis der Summe aus Estern und Fettalkoholen zur Lipidphase 20:1 bis 1:5 beträgt.

Die WO 01/19945 betrifft eine Tensid-Zusammensetzung, die Gemini-Tenside enthält und zudem einen C₆₋₄₀-Alkohol, ein Mono-, Di- und Triglycerid einer C₆₋₂₂-Carbonsäure sowie ein mit Milchsäure oder Citronensäure verestertes Derivat der Mono- und Diglyceride einer C₆₋₂₂-Carbonsäure. Neben dem Gemini-Tensid liegen zumindest ein C₆₋₄₀-Alkohol und/oder ein Mono-, Di- und Triglycerid einer C₆₋₂₂-Carbonsäure vor.

Die US 5 744 062 offenbart ein Emulgatorgemisch enthaltend Glycerylstearat, Stearylalkohol und Natriumstearoyllaktylat.

Aufgabe der vorliegenden Erfindung ist die Bereitstellung eines Emulgatorgemisches, das ein besonders gutes oder samtiges Hautgefühl aufweist, eine hohe Lagerstabilität auch bei erhöhten Temperaturen aufweist und zur Zubereitung einer Vielzahl von kosmetischen oder pharmazeutischen Zusammensetzungen geeignet ist. Insbesondere soll das Emulgatorgemisch zur Herstellung kosmetischer Zusammensetzungen zum Auftrag auf die menschliche Haut geeignet sein.

Die Aufgabe wird erfindungsgemäß gelöst durch ein Emulgatorgemisch, enthaltend
a) Glycerylstearat (Glyceryl Stearate) (A)
b) Glycerylstearatcitrat (Glyceryl Stearate Citrate) (B)
c) Stearylalkohol (Stearyl Alcohol) (C)
d) Natriumstearoyllactylat (Sodium Stearoyl Lactylate) (D).

Es wurde erfindungsgemäß gefunden, dass die Kombination von Glycerylstearat, Glycerylstearatcitrat, Stearylalkohol und Natriumstearoyllactylat zu einem

Emulgatorgemisch führt, das besonders angenehme Anwendungseigenschaften beim Auftrag auf die menschliche Haut zeigt und zudem eine sehr gute Stabilität, insbesondere Lagerstabilität bei erhöhten Temperaturen zeigt. Das Emulgatorgemisch kann in vorteilhafter Weise bei der Formulierung unterschiedlichster Emulsionen eingesetzt werden. Es ist beispielsweise möglich, Emulsionskonzentrate herzustellen, die sich in einfacher Weise mit Öl und Wasser auf eine Endkonzentration einstellen oder auffüllen lassen. Bevorzugt handelt es sich dabei um O/W-Emulsionen.

Die Bezeichnungen der Komponenten A bis D in Klammern entsprechen der INCI-Nomenklatur. Bei Glycerylstearat handelt es sich um einen Ester aus Glycerin und Stearinsäure, der üblicherweise als Emulgator eingesetzt wird. Die Verbindung ist aus einer Vielzahl von Quellen erhältlich.

Glycerylstearatcitrat ist ein neutralisierter Citronensäureester von Mono-Diglycerid der Stearinsäure. Das Mono-Diglycerid kann beispielsweise aus essbarem, vollständig hydriertem Palmöl erhalten werden. Der Gehalt an Citronensäure ist typischerweise 5 bis 20 Gew.-%, beispielsweise mindestens 13 Gew.-%. Der Säurewert beträgt vorzugsweise 5 bis 30, besonders bevorzugt 10 bis 25. Der Verseifungswert beträgt vorzugsweise 200 bis 300, besonders bevorzugt 220 bis 250. Der Jodwert beträgt besonders bevorzugt maximal 3. Die Verbindung hat vorzugsweise einen Tropfpunkt von etwa 64°C. Entsprechende Verbindungen sind unter anderem unter der Bezeichnung Grindsted®CITREM N 12 VEG Kosher von Danisco (www.danisco.com) erhältlich.

Stearylalkohol, auch als Octadecanol bezeichnet, weist ein Molekulargewicht von etwa 270 g/mol auf und wird typischerweise aus Kokosnuss- oder Palmkernöl gewonnen. Das Produkt kann beispielsweise unter der Bezeichnung Lanette® 18 von Cognis Deutschland GmbH & Co. KG erhalten werden. Das Produkt weist bevorzugt einen Hydroxylwert von 205 bis 210 und einen Verfestigungspunkt von 55 bis 58°C auf. Der Anteil an C₁₈-Ketten beträgt vorzugsweise mindestens 90, besonders bevorzugt mindestens 95 %.

Natriumstearoyllactylat (Sodium Stearoyl Lactylate) wird typischerweise aus raffinierten Fettsäuren pflanzlichen Ursprungs erhalten. Das Produkt hat bevorzugt einen Esterwert von 140 bis 200, besonders bevorzugt 150 bis 190, einen Säurewert von bevorzugt 50 bis 90, besonders bevorzugt 60 bis 8 und einen Jodwert von bevorzugt maximal 3, besonders bevorzugt maximal 2. Der Milchsäuregehalt beträgt vorzugsweise 25 bis 40 %, besonders bevorzugt 31 bis 34 %. Das Produkt ist beispielsweise unter der Bezeichnung Grindsted® SSL P 55 VEG Kosher von Danisco erhältlich.

Die einzelnen Komponenten A bis D können in beliebigen geeigneten Mengen im Emulgatorgemisch eingesetzt werden. Bevorzugt liegen 10 bis 90 Gew.-% der Komponenten A und B und 90 bis 10 Gew.-% der Komponenten C und D vor, bezogen auf die Gesamtmenge der Komponenten A bis D. Besonders bevorzugt ist ein Verhältnis von 30 bis 70 Gew.-%, insbesondere 40 bis 60 Gew.-%, beispielsweise 50 Gew.-% der Komponenten A und B und 70 bis 30 Gew.-%, insbesondere 60 bis 40 Gew.-%, beispielsweise 50 Gew.-% der Komponenten C und D.

Bezogen auf die Gesamtmenge der Komponenten A und B liegen bevorzugt 30 bis 80 Gew.-% der Komponente A und 70 bis 20 Gew.-% der Komponente B vor. Besonders bevorzugt liegen 50 bis 70 Gew.-%, beispielsweise 60 Gew.-% der Komponente A und 50 bis 30 Gew.-%, insbesondere 40 Gew.-% der Komponente B vor.

Bevorzugt liegen, bezogen auf die Gesamtmenge der Komponenten C und D, 10 bis 90 Gew.-% der Komponente C und 90 bis 10 Gew.-% der Komponente D vor. Besonders bevorzugt liegen 50 bis 80 Gew.-%, beispielsweise 50 Gew.-% der Komponente C und 50 bis 20 Gew.-%, beispielsweise 50 Gew.-% der Komponente D vor.

Beispielsweise liegen 30 Gew.-% Glycerylstearat, 20 Gew.-% Glycerylstearatcitrat, 25 Gew.-% Stearylalkohol und 25 Gew.-% Natriumstearoyllactylat vor.

Das Emulgatorgemisch wird bevorzugt in fester Form konfektioniert. Dabei kann es in jeder beliebigen geeigneten dreidimensionalen Form vorliegen, beispielsweise in Form von Kugeln, Pellets, Pastillen, Tabletten, Flocken.

Das erfindungsgemäße Emulgatorgemisch kann in beliebige geeignete Emulsionen eingebracht bzw. zu deren Herstellung verwendet werden. Die Erfindung betrifft somit auch eine Emulsion, enthaltend 0,5 bis 20 Gew.-%, bezogen auf die gesamte Emulsion, eines wie vorstehend beschriebenen Emulgatorgemisches. Dabei kann es sich um eine beliebige geeignete Emulsion handeln, beispielsweise um eine O/W-Emulsion, W/O-Emulsion oder eine multiple Emulsion vom Typ XIOIX oder XIOIY. Bevorzugt handelt es sich um eine O/W-Emulsion.

Bevorzugt enthält die Emulsion 1 bis 20 Gew.-%, besonders bevorzugt 2 bis 10 Gew.-%, insbesondere 3 bis 7 Gew.-% des beschriebenen Emulgatorgemisches.

Bei der Emulsion kann es sich um ein Emulsionskonzentrat handeln, das durch Zusatz von Öl- und Wasserphasen auf eine Endkonzentration verdünnt werden kann, oder es kann sich um die letztendlich herzustellende Emulsion handeln.

In der Emulsion können vorzugsweise zusätzlich zum Emulgatorgemisch ein oder mehrere zusätzliche O/W-Emulgatoren mit einem HLB-Wert > 7 vorliegen, wobei das Gewichtsverhältnis von Emulgatormischung zu O/W-Emulgatoren 1:9 bis 9:1 beträgt. Bezogen auf das Emulgatorgemisch und die zusätzlichen O/W-Emulgatoren liegen bevorzugt 50 bis 85 Gew.-% des Emulgatorgemisches, besonders bevorzugt 70 bis 80 Gew.-% des Emulgatorgemisches, beispielsweise 77 Gew.-% des Emulgatorgemisches und 50 bis 15 Gew.-%, besonders bevorzugt 30 bis 20 Gew.-%, beispielsweise 23 Gew.-% der zusätzlichen O/W-Emulgatoren vor. Als zusätzlicher O/W-Emulgator mit einem HLB-Wert > 7 wird bevorzugt Natriumlauroylsarcosinat (Sodium Lauroyl Sarcosinate) eingesetzt.

Das Emulsionskonzentrat oder die Emulsion kann jede beliebige geeignete Ölkomponente enthalten. Besonders bevorzugt wird ein Esteröl eingesetzt, insbesondere Caprylic Capric Triglyceride.

Das Emulsionskonzentrat oder die Emulsion enthält vorzugsweise zusätzlich ein Hydrocollloid, beispielsweise ein Dehydroxanthan Gum. Der Anteil an Hydrocolloid, bezogen auf das Emulsionskonzentrat, beträgt vorzugsweise 0,1 bis 5 Gew.-%.

Ferner kann ein Mittel zur Erhöhung der Kältestabilität zugesetzt werden, beispielsweise ein Polyol wie Glycerin. Der Anteil an Mittel zur Erhöhung der Kältestabilität im Emulsionskonzentrat beträgt vorzugsweise 1 bis 10 Gew.-%.

Zudem können Konservierungsstoffe wie Alkohole, beispielsweise Ethanol, zugesetzt werden. Der Anteil an Konservierungsstoffen beträgt bevorzugt 0,1 bis 10 Gew.-%, bezogen auf das Emulsionskonzentrat.

Als Caprylic Capric Triglyceride kann beispielsweise Miglyol 812 von Sasol eingesetzt werden. Als Sodium Lauroyl Sarcosinate kann beispielsweise Proteilan LS 9011 von Zschimmer & Schwartz verwendet werden. Als Dehydroxanthan Gum kann beispielsweise Amaze XT von National Starch eingesetzt werden. Eine beispielhafte Zusammensetzung des Emulsionskonzentrates weist 5,25 Gew.-% der erfindungsgemäßen Emulgatormischung, 26,35 Gew.-% Caprylic Capric Triglyceride (Esteröl), 52,95 Gew.-% entmineralisiertes Wasser, 5,25 Gew.-% Sodium Lauroyl Sarcosinate als Emulgator, 0,20 Gew.-% Dehydroxanthan Gum (Hydrocolloid), 5,00 Gew.-% Glycerin (zur Erhöhung der Kältestabilität) sowie 5,00 Gew.-% Ethanol (zur Konservierung) auf.

Beispielsweise kann auch Ethanol durch Kaliumsorbat/Natriumbenzoat ersetzt werden. Das Dehydroxanthan Gum kann alternativ durch Hydrocolloide wie Xanthan Gum, Arabic Gum, Cararageenane oder mikrokristalline Cellulose ersetzt werden. Anstelle des Esteröls können auch andere kosmetische Öle eingesetzt werden.

Der Anteil des erfindungsgemäßen Emulgatorgemisches im Emulsionskonzentrat beträgt bevorzugt 0,5 bis 20 Gew.-%, besonders bevorzugt 4 bis 6 Gew.-%.

Im Emulsionskonzentrat liegen, bezogen auf Ölphase einschließlich zusätzlichem O/W-Emulgator und wässrige Phase, bevorzugt 10 bis 95 Gew.-%, besonders bevorzugt 20 bis 80 Gew.-%, insbesondere 30 bis 35 Gew.-% Ölphase einschließlich O/W-Emulgator und 90 bis 5 Gew.-%, besonders bevorzugt 80 bis 20 Gew.-%, insbesondere 65 bis 70 Gew.-% wässrige Phase vor.

Das vorstehend beschriebene Emulsionskonzentrat bzw. die Emulsion kann als Emulsionskonzentrat zur Herstellung kosmetischer oder pharmazeutischer Zusammensetzungen eingesetzt werden. Bei kosmetischen Zusammensetzungen handelt es sich insbesondere um Hautkosmetik-Zusammensetzungen, bei pharmazeutischen Zusammensetzungen insbesondere um dermatologische Zusammensetzungen. Zur Herstellung der endgültigen Emulsionen ist es beispielsweise möglich, das Emulsionskonzentrat zunächst mit der erwünschten Menge an Ölkomponente aufzufüllen. Hierdurch steigt die Viskosität, und unter Scherung werden kleine Tröpfchen gebildet. Das zunächst turbulente Strömungsfeld geht dabei zunehmend in ein lamellares Strömungsfeld über. Anschließend kann die gewünschte Wassermenge in die so erhaltene Zusammensetzung eingebracht werden, wodurch die endgültige Emulsion erhalten wird.

Gemäß einer Ausführungsform der Erfindung werden keine endgültigen Emulsionen hergestellt und auch keine Emulsionskonzentrate eingesetzt, deren Viskosität bei 25°C kleiner ist als 2500 mPas, und die einen oder mehrere partiell neutralisierte Ester von Monoglyceriden und/oder Diglyceriden gesättigter Fettsäuren mit Citronensäure, einen oder mehrere Fettalkohole, gewählt aus der Gruppe der verzweigten und unverzweigten Alkylalkohole mit 12 bis 40 Kohlenstoffatomen, eine Wasserphase und von 2 bis 7,5 Gew.-% einer Lipidphase, bezogen auf das Gesamtgewicht der Zubereitungen, enthalten, wobei das Gewichtsverhältnis der Summen der Ester und Fettalkohole zur Lipidphase aus dem Bereich von 20:1 bis 1:5 gewählt wird. Damit sind gemäß einer Ausführungsform der Erfindung Emulsionen ausgeschlossen, die den in EP-B-1 049 452 geschützten Zusammensetzungen entsprechen.

Die Emulgatorgemische können zur Herstellung beliebiger Emulsionen eingesetzt werden. Bevorzugt ist die Herstellung solcher Emulsionen, wie sie in kosmetischen oder pharmazeutischen Zusammensetzungen eingesetzt werden. Dabei handelt es sich bevorzugt um hautkosmetische Zusammensetzungen oder dermatologische pharmazeutische Zusammensetzungen.

Die Ölphase kann beispielsweise ein Mineralöl, Silikonöl, Pflanzenöl oder Gemisch davon enthalten. Beispiele geeigneter pharmakologisch unbedenklicher Öle sind Silikonöle und Derivate davon, die linear oder zyklisch sein können, natürliche Esteröle wie Sonnenblumenöl, Traubenkernöl oder Olivenöl, synthetische Esteröle wie Neutralöle, die linear oder verzweigt sein können, Paraffinöle und Isoparaffinöle, Esteröle wie Citrate, Lactate, Aleate, Salicylate oder Cinnamate. Ferner können Lipide und Lipidähnliche Strukturen eingesetzt werden, beispielsweise Di- und Triglyceride der gesättigten, geradkettigen Fettsäuren mit 12 bis 30 Kohlenstoffatomen, wie Laurinsäure, Myristinsäure, Palmitinsäure, Stearinsäure, Arachinsäure, Behensäure, Lignocerinsäure, Cerotinsäure, Melesinsäure sowie deren Ester mit anderen gesättigten Fettalkoholen mit 4 bis 22, vorzugsweise 12 bis 22 Kohlenstoffatomen wie Laurylalkohol, Myristylalkohol, Cetylalkohol, Stearylalkohol, Arachidylalkohol, Behenylalkohol, gesättigten Wachsalkoholen mit 24 bis 30 Kohlenstoffatomen wie Lignocerylalkohol, Cerylalkohol, Cerotylalkohol, Myricylalkohol. Bevorzugt sind Di-, Triglyceride, Fettalkohole, deren Ester oder Ether, Wachse, Silikonöle wie Polydimethylsiloxan. Geeignete Glycerintrifettsäureester sind beispielsweise Glycerintrilaurat, Glycerintrimyristat, Glycerintripalmitat, Glycerintristearat oder Glycerintribehenat. Geeignete Wachse sind beispielsweise Cetylpalmitat und Cera Alba (gebleichtes Wachs).

Weitere geeignete Öl- und Lipidphasen sind in EP-A-1 000 603 in den Absätzen [0048] bis [0094] beschrieben.

Als wässrige Phase wird in der Regel Wasser eingesetzt. Die Wasserphase kann darüber hinaus Glycerin, Polyethylenglycol, Propylenglycol, Ethylenglycol und ähnliche Verbindungen sowie Derivate davon enthalten. Es können zudem wässrige Lösungen oder Mischungen von Wasser mit wassermischbaren Flüssigkeiten wie Glycerin, Alkoholen und anderen Polyolen eingesetzt werden. Mögliche Zusatzstoffe sind beispielsweise Glucose, Fructose, Mannose, Xylose, Mannit, Sorbit und Xylit. Ferner können Elektrolyte wie Natriumchlorid zugesetzt werden.

Bezogen auf die endgültige Emulsion werden vorzugsweise 0,5 bis 20 Gew.-%, besonders bevorzugt 1 bis 10 Gew.-%, insbesondere 3 bis 7 Gew.-% des Emulgatorgemisches eingesetzt.

Die Menge kann dabei nach den praktischen Erfordernissen, u. a. auch an die Lagerstabilität der Zusammensetzungen, gewählt werden.

Die notwendige Emulgatormenge hängt auch von der angestrebten Emulsionsendviskosität und von dem Gewichtsverhältnis zwischen Ölphase und Emulgator ab.

Die Erfindung betrifft auch eine kosmetische oder pharmazeutische Zusammensetzung, die eine wie vorstehend beschriebene Emulsion enthält. Es handelt sich bei der kosmetischen Zusammensetzung vorzugsweise um eine Hautpflegezusammensetzung und bei der pharmazeutischen Zusammensetzung um eine dermatologische Zusammensetzung. Die kosmetische oder pharmazeutische Zusammensetzung enthält das Emulgatorgemisch vorzugsweise in einer Menge im Bereich von 0,5 bis 20 Gew.-%, besonders bevorzugt 1 bis 10 Gew.-%, insbesondere 3 bis 7 Gew.-%, bezogen auf die gesamte Zusammensetzung.

Das erfindungsgemäße Emulgatorgemisch kann dabei in beliebigen geeigneten kosmetischen und pharmazeutischen Zusammensetzungen in Kombination mit weiteren Wirkstoffen eingesetzt werden.

Zusätzlich zum erfindungsgemäßen Emulgatorgemisch können auch weitere geeignete Emulgatoren eingesetzt werden.

Die Zusammensetzungen können viskositätserhöhende Stoffe wie Celluloseether und Celluloseester enthalten, beispielsweise Methylcellulose, Hydroxyethylcellulose, Hydroxypropylcellulose, Natriumcarboxymethylcellulose, sowie Polyvinylderivate wie Polyvinylalkohol, Polyvinylpyrrolidon, Polyvinylacetat, Alginate, Polyacrylate, Xanthane und Pektine.

Als Verdicker kommen zudem mikrokristalline Cellulose oder Carrageenane in Betracht.

Ferner können gegebenenfalls Polyole mitverwendet werden. Geeignete Polyole sind Propylenglycol, Butylenglycol, Ethylenglycol, Polyalkylenglycol, Glycerin, Polyglycerin, Glycoside, Sorbit, Mannit, Pentaeritrit, Dimethylolpropan oder Mischungen davon. Als Polyalkylenglycole können beispielsweise Polyethylenglycol und Polypropylenglycol eingesetzt werden. Ferner können Esterpolyole mitverwendet werden, die sich von Glycerin oder Alkylenglycolen ableiten können.

In den Zusammensetzungen kann das Spreit- und Haftvermögen durch Silikonzusatz wie Dimethicon, Cyclonethicon oder Silikonderivaten erhöht werden. Hierdurch kann auch die Wasserresistenz erhöht werden. Geeignete Silikonderivate sind dabei Dimethicon, Alkyl- und Aryl-substituierte Silikone, Silikon-Copolyole mit Alkylpolyglycosiden, Amino-substituierte Silikonöle, sowie andere modifizierte Silikonöle. Erfindungsgemäß zudem verwendbare kosmetische Hilfsstoffe sind beispielsweise Konservierungsmittel, Bakterizide, Parfume, Substanzen zum Verhindern des Schäumens, Farbstoffe, Pigmente, Verdickungsmittel, oberflächenaktive Substanzen, weich machende, anfeuchtende und/oder feucht haltende Substanzen oder Komplexbildner, die schädliche Metallionen durch Komplexbildung inaktivieren.

Geeignete Komplexbildner wie auch Antioxidantien sind in der EP-A-1 000 603 beschrieben. Für Komplexbildner kann auf die Absätze [0070] bis [0074] verwiesen werden. Für Antioxidantien kann auf die Absätze [0080] bis [0081] verwiesen werden.

Die erfindungsgemäßen Zusammensetzungen können ferner Duftstoffe und Aromen enthalten.

Unter Duftstoffen und Aromen werden erfindungsgemäß sowohl Duftöle (Flagrance) wie auch Aromastoffe (Flavour) verstanden. Es handelt sich hierbei um Riechstoffe, speziell Duftstoffe. Grundstoffe der Duftstoffe sind in der Regel etherische Öle, Blütenöle, Extrakte aus pflanzlichen und tierischen Drogen, aus Naturprodukten isolierte, chemisch veränderte (halbsynthetische) sowie rein synthetisch gewonnene Riechstoffe.

Die Duftstoffe und Aromen können dabei aus einer Vielzahl pflanzlicher Ausgangsmaterialien stammen. Beispielsweise können genannt werden: Blüten, beispielsweise von Lavendel, Rosen, Jasmin, Neroli; Stängel und Blätter, beispielsweise von Geranium, Patchouli, Petitgrain, Früchte wie Anis, Koriander, Kümmel, Wacholder; Fruchtschalen, beispielsweise von Agrumen wie Bergamotte, Zitronen, Orangen; Samen wie Macis, Angelika, Sellerie, Kardamom; Wurzeln wie Angelika, Costus, Iris, Calmus; Holz wie Sandel-, Guajak-, Zedern-, Rosenholz; Kräuter und Gräser wie Estragon, Limonengras, Salbei, Thymian; Nadeln und Zweige, beispielsweise von Fichten, Tannen, Kiefern, Latschen; Harze und Balsame, beispielsweise aus Galvanum, Elemi, Benzoe, Myrrhe, Olibanum, Opoponax.

Tierische Rohstoffe sind beispielsweise Ambra, Moschus, Zibet, Castoreum.

Beispiele halb synthetischer Riechstoffe sind Isoeugenol, Vanillin, Hydroxycitronellal, Citronellol, Geranylacetat, Jonone und Methyljonone. Die vollsynthetischen Riechstoffe oder Duftstoffe sind sehr vielfältig und orientieren sich häufig an natürlichen Stoffen. Für eine Beschreibung der Duftstoffe kann beispielsweise auf Römpp, Chemielexikon, 9. Auflage, Stichworte "Parfums", "Riechstoffe", "Duftstoffe", verwiesen werden. Weitere geeignete Duftstoffe und Aromen sind dem Fachmann bekannt.

Für den Einsatz in den erfindungsgemäßen Emulsionen geeignete kosmetische Wirkstoffe sind beispielsweise Antioxidantien.

Weitere geeignete Zusatzstoffe sind Panthenol und Panthotensäure, die aus dem medizinischen Bereich zur Wundheilung sowie aus Haarbehandlungsmitteln und Futterzusätzen bekannt sind.

Weiterhin geeignet sind kosmetische Wirkstoffe, die insbesondere oxidations- oder hydrolyseempfindlich sind wie beispielsweise Polyphenole. Hier seien genannt Catechine (wie Epicatechin, Epicatechin-3-gallat, Epigallocatechin, Epigallocatechin-3-gallat), Flavonoide (wie Luteolin, Apigenin, Rutin, Quercitin, Fisetin, Kaempherol, Rhametin), Isoflavone (wie Genistein, Daidzein, Glycitein, Prunetin), Cumarine (wie Daphnetin, Umbelliferon), Emodin, Resveratrol, Oregonin.

Geeignet sind Vitamine wie Retinol, Tocopherol, Ascorbinsäure, Riboflavin, Pyridoxin.

Geeignet sind ferner Gesamtextrakte aus Pflanzen, die u.a. obige Moleküle oder Molekülklassen enthalten.

Bei der erfindungsgemäßen Zusammensetzung kann es sich um ein Lichtschutzmittel handeln. Lichtschutzmittel sind dabei beispielsweise Sonnenöle, Sonnenmilch, Sonnencreme, Sonnenlotion, Sonnensprayöl oder Sonnenspray-Emulsion.

Bei den Wirkstoffen handelt es sich gemäß einer Ausführungsform der Erfindung um Lichtschutzfilter. Diese können als organische Lichtschutzfilter bei Raumtemperatur (25°C) in flüssiger oder fester Form vorliegen. Geeignete Lichtschutzfilter (UV-Filter) sind beispielsweise Verbindungen auf Basis von Benzophenon, Diphenylcyanacrylat oder p-Aminobenzoesäure. Konkrete Beispiele sind (INCI- oder CTFA-Bezeichnungen) Benzophenone-3, Benzophenone-4, Benzophenone-2, Benzophenone-6, Benzophenone-9, Benzophenone-1, Benzophenone-11, Etocrylene, Octocrylene, PEG-25 PABA, Phenylbenzimidazole Sulfonic Acid, Ethylhexyl Methoxycinnamate, Ethylhexyl Dimethyl PABA, 4-Methylbenzylidene Camphor, Butyl Methoxydibenzoylmethane, Ethylhexyl Salicylate, Homosalate sowie Methylene-Bis-Benzotriazolyl Tetramethylbutylphenol (2,2'-Methylen-bis-{6-(2H-benzoetriazol-2-yl)-4-(1,1,3,3-tetramethylbutyl)-phenol}, 2-Hydroxy-4-methoxybenzophenon-5-sulfonsäure und 2,4,6-Trianilino-p-(carbo-2'-ethylhexyl-1'-oxi)-1,3,5-triazin.

Weitere organische Lichtschutzfilter sind Octyltriazone, Avobenzone, Octylmethoxycinnamate, Octylsalicylate, Benzotriazole und Triazine. Für weitere UV-Absorber, insbesondere UVB-Filter und UVA-Filter kann auf EP-A-1 000 603, Absätze [0099] bis [0108] verwiesen werden.

In den Lichtschutzmitteln oder Sonnenschutzmitteln können ferner anorganische Pigmente wie Titandioxid- und Zinkoxid-Pigmente eingesetzt werden. Ferner können auch Ceroxid oder Zirkonoxid als UV-Absorber eingesetzt werden. Dabei kann es sich beispielsweise um Mikropigmente handeln. Die Mikropigmente oder Pigmente können zudem mit weiteren Zusatzstoffen oberflächlich beschichtet werden. In den Mikropigmenten beträgt die mittlere Teilchengröße vorzugsweise 5 bis 100 nm, besonders bevorzugt 10 bis 50 nm. Hydrophil gecoatete Mikropigmente können in die wässrige Phase eingebracht werden, hydrophob gecoatete Mikropigmente in die Ölphase.

In pharmazeutischen Zusammensetzungen kann eine Vielzahl geeigneter pharmakologisch aktiver Substanzen eingesetzt werden, die entweder in der Ölphase oder der wässrigen Phase löslich oder dispergierbar sind. Geeignete pharmakologische Wirkstoffe sind dem Fachmann bekannt.

Besonders bevorzugt handelt es sich bei den erfindungsgemäßen Zusammensetzungen um kosmetische Zusammensetzungen, die auf die Haut aufgetragen werden.

Die einzelnen Phasen der Emulsionen können noch übliche für die einzelnen Phasen bekannte Inhaltsstoffe aufweisen. Beispielsweise können die einzelnen Phasen weitere in diesen Phasen lösliche pharmazeutische oder kosmetische Wirkstoffe enthalten. Die wässrige Phase kann beispielsweise organische lösliche Lichtschutzfilter, hydrophil gecoatetes Mikropigment, Elektrolyte, Alkohole usw. enthalten. Einzelne oder alle der Phasen können zudem Feststoffe enthalten, die vorzugsweise ausgewählt sind aus Pigmenten oder Micropigmenten, Mikrosphären, Silikagel und ähnlichen Stoffen. Die Ölphase kann beispielsweise organisch modifizierte Tonmineralien, hydrophob gecoatete (Micro)Pigmente, organische öllösliche Lichtschutzfilter, öllösliche kosmetische Wirkstoffe, Wachse, Metallseifen wie Magnesiumstearat, Vaseline oder Gemische davon enthalten. Als (Micro)Pigmente können Titandioxid, Zinkoxid und Bariumsulfat sowie Wollastonit, Kaolin, Talk, Al₂O₃, Bismutoxidchlorid, micronisiertes Polyethylen, Glimmer, Ultramarin, Eosinfarben, Azofarbstoffe, genannt werden. Insbesondere Titandioxid oder Zinkoxid sind in der Kosmetik als Lichtschutzfilter gebräuchlich und lassen sich mittels der erfindungsgemäßen Emulsionen besonders glatt und gleichmäßig auf die Haut auftragen. Mikrosphären oder Silikagel können als Träger für Wirkstoffe eingesetzt werden, und Wachse können beispielsweise als Grundlage für Polituren verwendet werden.

Die Emulsionen oder Dispersionen können in Abhängigkeit von der Zusammensetzung, vom Phasenvolumenverhältnis und dem gegebenenfalls vorhandenen Feststoffanteil als feste oder fließfähige Emulsionen oder Dispersionen hergestellt werden und vorliegen. Es handelt sich dabei um sehr stabile Emulsionen oder Dispersionen, die unter normalen Handhabungsbedingungen eine hohe Langzeitstabilität aufweisen. Sie erfüllen insbesondere die üblichen Stabilitätsanforderungen im Temperaturbereich von -5 °C bis + 45 °C. Die in der Emulsion oder Dispersion vorliegenden Tröpfchen oder Partikel sind dabei sehr beständig, weshalb die Emulsionen oder Dispersionen insbesondere als Träger für viele Arten von Wirkstoffen geeignet sind.

Die mit Hilfe der genannten Emulgatorgemische hergestellten Emulsionen oder Dispersionen können durch einen einfachen Mischvorgang unter Rühren, vorzugsweise unter höheren Scherkräften mittels Rotor/Stator-System hergestellt werden.

Die erfindungsgemäßen Zusammensetzungen können durch Eintrag entsprechender Wirkstoffe an die jeweilige Anwendung angepasst werden. Beispiele geeigneter, weiterer Zusatzstoffe sind Nährstoffe, Vitalstoffe, Mineralstoffe, Vitamine, Spurenelemente, biologisch wirksame Stoffe und therapeutisch wirksame Stoffe. Beispielsweise können Vitamine und Mineralstoffe enthalten sein.

Die Herstellung der Zusammensetzungen erfolgt nach bekannten Verfahren durch Vermischen der Inhaltsstoffe. Die Ausbildung der Emulsion wird typischerweise durch Energieeintrag mittels Homogenisierung, vorzugsweise durch ein Rotor/Stator-System erreicht.

Die Erfindung wird durch die nachfolgenden Beispiele näher erläutert.

### Beispiele

### Beispiel 1

Es wurden fünf unterschiedliche Emulgatormischungen durch Vermischen der nachstehend angegebenen Mengen der Komponenten hergestellt. Die Mischungen V1 bis V4 sind Vergleichsmischungen, während Mischung 5 eine erfindungsgemäße Mischung ist, die die vier erfindungsgemäß eingesetzten Emulgatorbestandteile aufweist.

Die Zusammensetzungen sind in der nachstehenden Tabelle 1 angegeben.

**Tabelle 1**

| **Mischung** | | | **V1** | **V2** | **V3** | **V4** | **5** |
|---|---|---|---|---|---|---|---|
| **Phase A** | **CTFA/INCI** | **Lieferant** | **Gew.- %** | **Gew.- %** | **Gew.- %** | **Gew.- %** | **Gew.- %** |
| Lanette 18 | Stearyl Alcohol | Cognis | 50,00 | 40,00 | 0,00 | 40,00 | 25,00 |
| Dermofeel SL | Sodium Stearoyl Lactylate | Straetmans | 50,00 | 0,00 | 0,00 | 0,00 | 25,00 |
| Sympatens GMS | Glyceryl Stearate | Kolb | 0,00 | 40,00 | 60,00 | 30,00 | 30,00 |
| Imwitor 560 | Sodium Lauroyl Lactylate | Sasol | 0,00 | 20,00 | 0,00 | 10,00 | 0,00 |
| Imwitor 372 | Glyceryl Stearate Citrate | Sasol | 0,00 | 0,00 | 40,00 | 20,00 | 20,00 |
| **total** | | | **100,00** | **100,00** | **100,00** | **100,00** | **100,00** |

### Beispiel 2

Mit den Emulgatormischungen aus Beispiel 1 wurden unterschiedliche Vergleichsrezepturen V1 bis V4 und eine erfindungsgemäße Rezeptur 5 hergestellt. Die Zusammensetzungen sind in der nachstehenden Tabelle 2 angegeben. Zur Herstellung wurden zunächst die Phasen A und B auf 70°C erhitzt, sodann wurde Phase A zu Phase B gegeben, und es wurde für eine Minute homogenisiert. Sodann wurde bei etwa 40°C nachhomogenisiert. Nach Abkühlen auf Raumtemperatur wurde Phase C zugegeben.

**Tabelle 2**

| **Rezeptur** | **CTFA/INCI** | **Lieferant** | **V1** | **V2** | **V3** | **V4** | **Rez 5** |
|---|---|---|---|---|---|---|---|
| **Phase A** | | | **Gew.- %** | **Gew.- %** | **Gew.- %** | **Gew.- %** | **Gew.- %** |
| V1 | | | **5,50** | 0,00 | 0,00 | 0,00 | 0,00 |
| V2 | | | 0,00 | **5,50** | 0,00 | 0,00 | 0,00 |
| V3 | | | 0,00 | 0,00 | **5,50** | 0,00 | 0,00 |
| V4 | | | 0,00 | 0,00 | 0,00 | **5,50** | 0,00 |
| 5 | | | 0,00 | 0,00 | 0,00 | 0,00 | **5,50** |
| Miglyol 812 | Caprylic Capric Triglyceride | Sasol | 5,00 | 5,00 | 5,00 | 5,00 | 5,00 |
| Palmil O | Octyl Pamiltate | Undesa | 6,00 | 6,00 | 6,00 | 6,00 | 6,00 |
| Blanova Sweet Almond Oil | Prunus Amygdalus Dulcis (Sweet Almond) Oil | S.Black | 3,00 | 3,00 | 3,00 | 3,00 | 3,00 |

| **Phase B** | | | | | | | |
|---|---|---|---|---|---|---|---|
| demin.water | demin.water | | 70,80 | 70,80 | 70,80 | 70,80 | 70,80 |
| Ectoin | Ectoin | Merck | 0,50 | 0,50 | 0,50 | 0,50 | 0,50 |
| Glycerin | Glycerin | Merck | 4,00 | 4,00 | 4.00 | 4,00 | 4,00 |
| Amaze XT | Dehydroxanthan Gum | National Starch | 0,20 | 0,20 | 0,20 | 0,20 | 0,20 |

| **Phase C** | | | | | | | |
|---|---|---|---|---|---|---|---|
| Ethanol | Ethanol | Merck | 5,00 | 5,00 | 5.00 | 5,00 | 5,00 |
| **total** | | | **100,00** | **100,00** | **100,00** | **100,00** | **100,00** |
| **pH-Wert** | | | 5,2 | 6,8 | 4,8 | 6,4 | 5,1 |

Die Konsistenz der Testemulsionen wurde per Hand ermittelt. Das Hautgefühl wurde von mehreren Testpersonen bestimmt. Das Aussehen wurde visuell überprüft. Durch Mikroskopie wurde die Homogenität der Emulsionen bestimmt, und es wurde ermittelt, ob eine flüssig-kristalline Gel-Netzwerk-Struktur erhalten wurde. Nur für die erfindungsgemäße Rezeptur 5 wurde eine ideale homogene liquid-kristalline Gel-Netzwerk-Struktur erhalten. Ebenfalls wurde nur für die Rezeptur 5 ein samtiges Hautgefühl erreicht. Zudem wurde für die Rezeptur 5 eine hohe Viskosität bestimmt.

Sodann wurden die Rezepturen einem Stabilitätstest bei einer Lagerung bei Raumtemperatur (RT) und 40°C unterzogen. Zudem wurden die Rezepturen fünf Tau-Gefrier-Zyklen (TGC) unterworfen, wobei abwechselnd bei -18°C und +40°C gelagert wurde, mit einer Temperaturänderung jeweils innerhalb von 24 Stunden. Die Ergebnisse sind nachstehend in Tabelle 3 und 4 angegeben.

**Tabelle 3**

| | **Rez 1** | **Rez 2** | **Rez 3** | **Rez 4** | **Rez 5** |
|---|---|---|---|---|---|
| Konsistenz | viel | wenig | mittel | mittel | viel |
| Weichheit/Samtigkeit | mittel | wenig | wenig | mittel | viel |
| Glanz | viel | mittel | wenig | wenig | viel |
| Homogenität | wenig | mittel | wenig | wenig | viel |
| Mikroskopie/ Teilchengrößenverteilung | homogen | homogen | polydispers | homogen | homogen |

| **Rheologie** | | | | | |
|---|---|---|---|---|---|
| Viskosität [mPas] bei 1/s | 71000 | 7500 | 38000 | 42000 | 72000 |

**Tabelle 4**

| Rezeptur-Nr. | | **V1** | **V2** | **V3** | **V4** | **Rez 5** |
|---|---|---|---|---|---|---|
| **RT** | | | | | | |
| 1-3 Tag(e) | | 1 | 1 | 1 | 1 | 1 |
| 1 Woche | | 1 | 1 | 1 | 1 | 1 |
| 2 Wochen | | 1 | 1 | 1 | 1 | 1 |
| 3 Wochen | | 1 | 1 | 1 | 1 | 1 |
| 4 Wochen | | 1 | 1 | 1 | 1 | 1 |
| 6 Wochen | | 1 | 1 | 1 | 1 | 1 |
| 2 Monate | | 1 | 1 | 1 | 1 | 1 |
| 3 Monate | | Agglomerate | | | | |
| **40°C** | | | | | | |
| 1-3 Tag(e) | | 1 | 1 | 1 | 1 | 1 |
| 1 Woche | | 1 | 1 | 1 | 1 | 1 |
| 2 Wochen | | 1 | 1 | 1 | 1 | 1 |
| 3 Wochen | | 1 | 1, I.schlierig | 1 | 1 | 1 |
| 4 Wochen | | 1 | 1,I.schlierig | 1 | 1 | 1 |
| 6 Wochen | | 1 | 1,I.quarkig | 1 | 1 | 1 |
| 2 Monate | | 4/W 3% | 3/W | 3/W | 3/W | 1 |
| 3 Monate | | - | - | - | - | 1 |
| **TGC** | | | | | | |
| 1X | | 1 | 1 | 1 | 1 | 1 |
| 2X | | 1 | 1 | 1 | 1 | 1 |
| 3X | | 1 | 1 | 1 | 1 | 1 |
| 4X | | 1 | 1 | 1 | 1 | 1 |
| 5X | | 1 | 1 | 1 | 1 | 1 |

| | | | | | | |
|---|---|---|---|---|---|---|
| 1=ok 2=nicht homogen 3=Phasentrennung, nicht messbar 4=Phasentrennung, messbar 5=vollständige Phasentrennung | | | | | | |

Aus den Ergebnissen geht hervor, dass die Rezeptur 5 das beste Lagerverhalten zeigt. Insbesondere bei einer Lagertemperatur von 40°C blieb die Testemulsion über zwei und drei Monate unverändert erhalten.

### Beispiel 3

Mit der Emulgatormischung 5 aus Beispiel 1 wurden unterschiedliche Testemulsionen mit unterschiedlicher Emulgator-Konzentration hergestellt. Die Herstellung erfolgte wie in Beispiel 2 beschrieben, wobei der pH-Wert abschließend auf 7 eingestellt wurde. Die Zusammensetzungen sind in der nachstehenden Tabelle 5 angegeben.

**Tabelle 5**

| **Rezeptur** | **CTFA/INCI** | | **Lieferant** | **Rez 1** | **Rez 2** | **Rez 3** | **Rez 4** |
|---|---|---|---|---|---|---|---|
| **Phase A** | | | | **Gew.- %** | **Gew.- %** | **Gew.- %** | **Gew.- %** |
| 5 | Glyceryl Stearate, Stearyl alcohol, Sodium Stearoyl lactylate, Glyceryl Stearate Citrate | | / | **1,00** | **3,00** | **5,50** | **7,50** |
| Miglyol 812 | Caprylic Capric Triglyceride | | Sasol | 5,00 | 5,00 | 5,00 | 5,00 |
| Palmil O | Octyl Pamiltate | | Undesa | 6,00 | 6,00 | 6,00 | 6,00 |
| Blanova Sweet Al-mond Oil | Prunus Amygdalus Dulcis (Sweet Almond) Oil | | S.Black | 3,00 | 3,00 | 3,00 | 3,00 |

| **Phase B** | | | | | | | |
|---|---|---|---|---|---|---|---|
| demin.water | | | | 75,30 | 73,30 | 70,80 | 68,80 |
| Ectoin | Ectoin | | Merck | 0,50 | 0,50 | 0,50 | 0,50 |
| Glycerin | Glycerin | | Merck | 4,00 | 4,00 | 4,00 | 4,00 |
| Amaze XT | Dehydroxanthan Gum | | National Starch | 0,20 | 0,20 | 0,20 | 0,20 |

| **Phase B** | | | | | | | |
|---|---|---|---|---|---|---|---|
| Ethanol | Ethanol | | Merck | 5,00 | 5,00 | 5,00 | 5,00 |
| **total** | | | | **100,00** | **100,00** | **100,00** | **100,00** |
| **Rheologie** | | | | | | | |
| Fließpunkt [mPa] | | | | wasser dünn | 612 | 14285 | 24489 |
| Viskosität [mPas] bei 1/s | | | | | 15600 | 70000 | 100000 |

Alle Testemulsionen überstehen fünf Tau-Gefrier-Zyklen unverändert. Für die Rezepturen 2 bis 4 wurde eine Lagerstabilität von drei Monaten bei 40°C und von drei bis vier Wochen bei 50°C bestimmt.

### Beispiel 4

Mit dem erfindungsgemäßen Emulgatorgemisch 5 aus Beispiel 1 wurden Testemulsionen mit unterschiedlichen Ölphasen hergestellt. Die Zusammensetzungen sind in der nachfolgenden Tabelle 6 angegeben. Die Herstellung erfolgte wie in Beispiel 3 beschrieben.

Die Rezeptur 1 enthält polare Ölkomponenten, Rezeptur 2 überwiegend unpolare Ölkomponenten, Rezeptur 3 eine gemischt-polare Ölphase und Rezeptur 4 ebenfalls eine gemischt-polare Ölphase und zudem Cyclomethicone.

**Tabelle 6**

| **Rezeptur** | | | **Rez 1** | **Rez 2** | **Rez 3** | **Rez 4** |
|---|---|---|---|---|---|---|
| | **CTFA/INCI** | **Lieferant** | **Gew.- %** | **Gew.- %** | **Gew.- %** | **Gew.- %** |
| **Phase A** | | | | | | |
| 5 | Glyceryl Stearate, Stearyl alcohol, Sodium Stearoyl lactylate, Glyceryl Stearate Citrate | / | 3,00 | 3,00 | 3,00 | 3,00 |
| Miglyol 812 | Caprylic Capric Triglyceride | Sasol | 5,00 | 0,00 | **2,50** | 1,70 |
| Lexol-IPP | Isopropyl Palmitate | Inolex | 6,00 | **5,00** | **2,50** | 2,00 |
| Blanova Sweet Almond Oil | Prunus Amygdalus Dulcis (Sweet Almond) Oil | S.Black | 5,00 | 0,00 | **2,50** | 1,70 |
| Isohexadecane | Isohexadecane | Lanxess | 0,00 | **0,00** | 3,00 | 1,70 |
| Squalane | Squalane | Worlee | 0,00 | **6,00** | 3,00 | 2,00 |
| Merkur White Oil Pharma 40 PB | Paraffinum Liquidum | Merkur | 0,00 | **2,50** | 1,25 | 0,80 |
| Vara AB | Petrolatum | Merkur | 0,00 | **2,50** | 1,25 | 0,80 |
| KF995 | Cyclomethicone | Shin-Etsu | 0,00 | 0,00 | 0,00 | **5,30** |

| **Phase B** | | | | | | |
|---|---|---|---|---|---|---|
| demin.water | demin.water | Merck | 71,80 | 71,80 | 71,80 | 71,80 |
| Glycerin | Glycerin | Merck | 4,00 | 4,00 | 4,00 | 4,00 |
| Amaze XT | Dehydroxanthan Gum | National Starch | 0,20 | 0,20 | 0,20 | 0,20 |

| **Phase C** | | | | | | |
|---|---|---|---|---|---|---|
| Ethanol | Ethanol | Merck | 5,00 | 5,00 | 5,00 | 5,00 |
| **total** | | | **100,00** | **100,00** | **100,00** | **100,00** |
| **Rheologie** | | | | | | |
| Fließpunkt [mPa] | | | 1224 | 6122 | 3061 | 3061 |
| Viskosität [mPas] bei 1/s | | | 14600 | 38000 | 25000 | 24600 |

Im Stabilitätstest zeigen alle Testemulsionen bei Raumtemperatur eine Lagerstabilität von mindestens 6 Wochen, bei 40°C von 2 Monaten, bei 50°C von 4 bis 6 Wochen. Nur die erste Testemulsion zeigt bei 50°C eine Stabilität von mindestens einer Woche. Alle Emulsionen durchlaufen fünf Tau-Gefrier-Zyklen ohne Veränderung.

### Beispiel 5

Es wurde eine sprühbare Formulierung unter Verwendung der erfindungsgemäßen Emulgatormischung aus Beispiel 1 hergestellt. Die Herstellung erfolgte wie in Beispiel 2 beschrieben, wobei abschließend Phase D zugegeben wurde.

**Tabelle 7**

| | **CTFA/INCI** | **Lieferant** | **Gew.- %** |
|---|---|---|---|
| **Phase A** | | | |
| 5 | Glyceryl Stearate, Stearyl alcohol, Sodium Stearoyl lactylate, Glyceryl Stearate Citrate | / | 1,00 |
| Miglyol 812 | Caprylic Capric Triglyceride | Sasol | 10,000 |
| Palmil O | Octyl Palmitate | Undesa | 11,000 |
| Blanova Sweet Almond Oil | Prunus Amygdalus Dulcis (Sweet Almond) Oil | S.Black | 3,000 |

| **Phase B** | | | |
|---|---|---|---|
| Montanov 68 | Cetearyl Glucoside | Seppic | **2,00** |
| demin.water | demin.water | | 8,00 |
| Glycerin | Glycerin | Merck | 5,00 |

| **Phase C** | | | |
|---|---|---|---|
| demin.water | | | 29,50 |
| Avicel PC 611 (4%) | Water, Microcrystalline Cellulose, Cellulose Gum | FMC | 25,00 |
| Ectoin | Ectoin | Merck | 0,50 |

| **Phase D** | | | |
|---|---|---|---|
| Ethanol | Methanol | Merck | 5,00 |
| **total** | | | **100,00** |
| **pH-Wert** | | | 5,7 |
| **Rheologie** | | | |
| Fließpunkt [mPa] | | | 1020 |
| Viskosität [mPas] bei 1/s | | | 11000 |

Die Emulsion zeigt bei 40°C eine Lagerstabilität von mindestens drei Monaten, bei 50°C von zwei Monaten, und sie durchläuft fünf Tau-Gefrier-Zyklen ohne Veränderung.

### Beispiel 6

Es wurden unterschiedliche Testemulsionen mit der erfindungsgemäßen Emulgatormischung aus Beispiel 1 hergestellt, wobei unterschiedliche Konservierungsmittel eingesetzt wurden. Die Zusammensetzungen sind in der nachstehenden Tabelle 8 aufgeführt.

Die Herstellung der Emulsionen erfolgte wie in Beispiel 3 beschrieben.

**Tabelle 8**

| **Rezeptur** | | | **Rez 1** | **Rez 2** | **Rez 3** |
|---|---|---|---|---|---|
| | **CTFA/INCI** | **Lieferant** | **Gew.- %** | **Gew.- %** | **Gew.- %** |
| **Phase A** | | | | | |
| 5 | Glyceryl Stearate, Stearyl alcohol, Sodium Stearoyl lactylate, Glyceryl Stearate Citrate | / | 3,00 | 3,00 | 3,00 |
| Miglyol 812 | Caprylic Capric Triglyceride | Sasol | 2,50 | 2,50 | 2,50 |
| Lexol-IPP | Isopropyl Palmitate | Inolex | 2,50 | 2,50 | 2,50 |
| Blanova Sweet Almond Oil | Prunus Amygdalus Dulcis (Sweet Almond) Oil | S.Black | 2,50 | 2,50 | 2,50 |
| Isohexadecane | Isohexadecane | Lanxess | 3,00 | 3,00 | 3,00 |
| Squalane | Squalane | Worlee | 3,00 | 3,00 | 3,00 |
| Merkur White Oil Pharma 40 PB | Paraffinum Liquidum | Merkur | 1,25 | 1,25 | 1,25 |
| Vara AB | Petrolatum | Merkur | 1,25 | 1,25 | 1,25 |

| **Phase B** | | | | | |
|---|---|---|---|---|---|
| demin.water | Ectoin | Merck | 71,80 | 71,60 | 75,80 |
| Glycerin | Glycerin | Merck | 4,00 | 4,00 | 4,00 |
| Amaze XT | Dehydroxanthan Gum | National Starch | 0,20 | 0,20 | 0,20 |
| Paratexin NAP | Phenethyl Alcohol, Gaprylyl Glycol | S.Black Ltd. | 0,00 | 0,00 | **1,00** |

| **Phase C** | | | | | |
|---|---|---|---|---|---|
| Kaliumsorbat | Potassium Sorbate | Merck | 0,00 | **0,05** | 0,00 |
| Natriumbenzoate | Sodium Benzoate | Merck | 0,00 | **0,15** | 0,00 |
| demin.water | demin.water | | 0,00 | 5,00 | 0,00 |

| **Phase C** | | | | | |
|---|---|---|---|---|---|
| Ethanol | Ethanol | Merck | 5,00 | 0,00 | 0,00 |
| **total** | | | **100,00** | **100,00** | **100,00** |

Alle Emulsionen waren für fünf Tau-Gefrier-Zyklen stabil und sowohl bei Raumtemperatur als auch bei erhöhten Temperaturen lagerstabil.

### Beispiel 7

Es wurde ein Emulsionskonzentrat unter Verwendung des erfindungsgemäßen Emulgatorgemisches aus Bespiel 1 hergestellt. Die Herstellung erfolgte wie in Beispiel 2 beschrieben. Die Zusammensetzung ist in der nachstehenden Tabelle 9 angegeben.

**Tabelle 9**

| | **CTFA/INCI** | **Lieferant** | **Gew.- %** |
|---|---|---|---|
| **Phase A** | | | |
| 5 | Glyceryl Stearate, Stearyl alcohol, Sodium Stearoyl lactylate, Glyceryl Stearate Citrate | | **5,80** |
| Miglyol 812 | Caprylic Capric Triglyceride | Sasol | 11,60 |
| Palmil O | Octyl Palimtate | Undesa | 14,60 |
| Blanova Sweet Almond Oil | Prunus Amygdalus Dulcis (Sweet Almond) Oil | S.Black | 2,90 |

| **Phase B** | | | |
|---|---|---|---|
| Protelan LS 9011 | Sodium Lauroyl Sarcosinate | Zschimmer&Schwarz | 11,60 |
| demin.water | demin.water | | 41,50 |
| Glycerin | Glycerin | Merck | 7,00 |

| **Phase C** | | | |
|---|---|---|---|
| Ethanol | Ethanol | Merck | 5,00 |
| **total** | | | **100,00** |

### Beispiel 8

Aus dem Emulsionskonzentrat aus Beispiel 7 wurde eine Sprühformulierung hergestellt, indem bei Raumtemperatur Phase B zu Phase A gegeben wurde und für eine Minute homogenisiert wurde. Sodann wurde Phase C eingearbeitet, anschließend Phase D.

**Tabelle 10**

| **Phase A** | **CTFA/INCI** | **Lieferant** | **Gew.- %** |
|---|---|---|---|
| | | | |
| Konzentrat aus Bsp. 7 | | | **17,20** |

| **Phase B** | | | |
|---|---|---|---|
| Miglyol 812 | Caprylic Capric Triglyceride | Sasol | 10,00 |
| Palmil O | Octyl Palimtate | Undesa | 11,00 |
| Blanova Sweet Almond Oil | Prunus Amygdalus Dulcis (Sweet Almond) Oil | S.Black | 3,00 |

| **Phase C** | | | |
|---|---|---|---|
| demin.water | demin.water | | 24,80 |
| Glycerin | Glycerin | Merck | 4,00 |
| Avicel PC 611 (4%) | water, Microcrystalline Cellulose, Cellulose Gum | FMC | 25,00 |

| **Phase D** | | | |
|---|---|---|---|
| Ethanol | Ethanol | Merck | 5,00 |
| **total** | | | **100,00** |

### Beispiel 9

Mit dem Emulsionskonzentrat aus Beispiel 7 wurden Sprühformulierungen hergestellt. Es handelte sich um eine After-Sun-Formulierung (Rezeptur 1), um eine Dehydroxyaceton-enthaltende Formulierung (Rezeptur 2) und um eine Formulierung mit dem Sonnenschutzfaktor 20 (Rezeptur 3). Die Zusammensetzungen sind in der nachfolgenden Tabelle 11 angegeben. Zur Herstellung wurden die Phasen A und B getrennt auf etwa 70°C erhitzt. Sodann wurde Phase A zu Phase B gegeben, und es wurde für etwa eine Minute homogenisiert. Nach Abkühlen auf Raumtemperatur wurde Phase C zugegeben, und anschließend mit Phase D neutralisiert (Rezeptur 2). Daraufhin wurden die Phasen E und F eingearbeitet.

**Tabelle 11**

| **Rezeptur** | | | **Rez 1** | **Rez 2** | **Rez 3** |
|---|---|---|---|---|---|
| **Phase A** | **CTFA/INCI** | **Lieferant** | **Gew.- %** | **Gew.- %** | **Gew.- %** |
| Konzentrat aus Bsp. 7 | Water, Capric Caprylic Triglyceride, Glycerin, Ethanol, Sodium Lauroyl Sarcosinate, Glyceryl Stearate Citrate, Glyceryl Stearate, Stearyl Alcohol, Sodium Stearoyl Lactylate | | 19,00 | 19,00 | 19,00 |

| Phase B | | | | | |
|---|---|---|---|---|---|
| Miglyol 812 | Caprylic Capric Triglyceride | Sasol | 4,00 | 5,00 | 2,00 |
| Eusolex OCR | Octocrylene | Merck | 0,00 | 0,00 | 10,00 |
| Eusloxex 9020 | Butyl Methoxydibenzoyl methane | Merck | 0,00 | 0,00 | 2,00 |
| Jojobaöl Natural Gold | Simmondsia Chinensis Seed Oil | Biochemica | 4,00 | 4,00 | 0,00 |
| Blanova Sweet Almond Oil | Prunus Amygdalus Dulcis (Sweet Almond) Oil | S.Black | 3,00 | 0,00 | 2,00 |
| Shea Butter | Shea Butter | | 1,00 | 1,00 | 1,00 |
| Blanova Vitamin E acetate | Tocopheryl Acetate | S.Black | 0,50 | 0,50 | 0,50 |

| Phase C | | | | | |
|---|---|---|---|---|---|
| Eusloxe T-AVO | Titanium Dioxide, Silica | Merck | 0,00 | 0,00 | 4,00 |
| demin.water | demin.water | | 30,50 | 20,30 | 29,50 |
| Avicel PC 611 (4%) | Water, Microcrystalline Cellulose, Cellulose Gum | FMC | 25,00 | 25,00 | 25,00 |

| Phase D | | | | | |
|---|---|---|---|---|---|
| Citric Acid (40%) | Citric Acid | Merck | 0,00 | 0,20 | 0,00 |

| Phase E | | | | | |
|---|---|---|---|---|---|
| DHA | Dehydroxyaceton | Merck | 0,00 | 3,50 | 0,00 |
| Erythrulose | Erythrulose | Pentapharm | 0,00 | 1,50 | 0,00 |
| water | water | | 0,00 | 10,00 | 0,00 |

| Phase F | | | | | |
|---|---|---|---|---|---|
| ALPAFLOR CALENDULA GC | Glycerin, Water, Calendula Officinalis Flower Extract, preservative | Pentapharm | 5,00 | 0,00 | 0,00 |
| Actiphyte of Cucumber | Propylene Glycol, Water, Cucumis Sativus (Cucumber) Extract, preservative | Active Organics | 0,00 | 5,00 | 0,00 |
| Blanova Aloe Vera Gel 10* | water, Aloe Vera | S.Black | 3,00 | 0,00 | 0,00 |
| Ethanol | Ethanol | Merck | 5,00 | 5,00 | 5,00 |
| total | | | 100,00 | 100,00 | 100,00 |
| Rheologie | | | | | |
| Fließpunkt [mPa] | | | 102 | 102 | 102 |
| Viskosität [mPas] bei 1/s | | | 850 | 1300 | 1600 |

Die Rezepturen 1 und 3 zeigen eine Lagerstabilität bei 40°C von mindestens 4 Wochen bei 40 und 50°C. Alle Formulierungen durchlaufen 5 Tau-Gefrier-Zyklen unverändert.

## Patentansprüche

1. Emulgatorgemisch, enthaltend
a) Glycerylstearat (A)
b) Glycerylstearatcitrat (B)
c) Stearylalkohol (C)
d) Natriumstearoyllactylat (D).

2. Emulgatorgemisch nach Anspruch 1, **dadurch gekennzeichnet, dass** 10 bis 90 Gew.-% der Komponenten A und B und 90 bis 10 Gew.-% der Komponenten C und D vorliegen, bezogen auf die Gesamtmenge der Komponenten A bis D.

3. Emulgatorgemisch nach Anspruch 1 oder 2, **dadurch gekennzeichnet, dass** 30 bis 80 Gew.-% der Komponente A und 70 bis 20 Gew.-% der Komponente B vorliegen, bezogen auf die Gesamtmenge der Komponenten A und B.

4. Emulgatorgemisch nach einem der Ansprüche 1 bis 3, **dadurch gekennzeichnet, dass** 10 bis 90 Gew.-% der Komponente C und 90 bis 10 Gew.-% der Komponente D vorliegen, bezogen auf die Gesamtmenge der Komponenten C und D.

5. Emulsion, enthaltend 0,5 bis 20 Gew.-%, bezogen auf die gesamte Emulsion, eines Emulgatorgemisches nach einem der Ansprüche 1 bis 4.

6. Emulsion nach Anspruch 5, **dadurch gekennzeichnet, dass** es sich um eine O/W-Emulsion handelt.

7. Emulsion nach Anspruch 5 oder 6, **dadurch gekennzeichnet, dass** zusätzlich zum Emulgatorgemisch ein oder mehrere O/W-Emulgatoren mit einem HLB-Wert > 7 vorliegen, wobei das Gewichtsverhältnis von Emulgatormischung zu zusätzlichen O/W-Emulgatoren 1:9 bis 9:1 beträgt.

8. Emulsion nach Anspruch 7, **dadurch gekennzeichnet, dass** als zusätzlicher Emulgator mit einem HLB-Wert > 7 Natriumlauroylsarcosinat eingesetzt wird.

9. Emulsion nach einem der Ansprüche 5 bis 8, **dadurch gekennzeichnet, dass** die Ölkomponente Caprylic Capric Triglyceride enthält.

10. Verwendung einer Emulsion nach einem der Ansprüche 5 bis 9 als Emulsionskonzentrat zur Herstellung kosmetischer oder pharmazeutischer Zusammensetzungen.

## Claims

1. Emulsifier mixture comprising
a) glyceryl stearate (A)
b) glyceryl stearate citrate (B)
c) stearyl alcohol (C)
d) sodium stearoyl lactylate (D).

2. Emulsifier mixture according to Claim 1, **characterized in that** 10 to 90% by weight of components A and B and 90 to 10% by weight of components C and D are present, based on the total amount of components A to D.

3. Emulsifier mixture according to Claim 1 or 2, **characterized in that** 30 to 80% by weight of component A and 70 to 20% by weight of component B are present, based on the total amount of components A and B.

4. Emulsifier mixture according to one of Claims 1 to 3, **characterized in that** 10 to 90% by weight of component C and 90 to 10% by weight of component D are present, based on the total amount of components C and D.

5. Emulsion comprising 0.5 to 20% by weight, based on the total emulsion, of an emulsifier mixture according to one of Claims 1 to 4.

6. Emulsion according to Claim 5, **characterized in that** it is an O/W emulsion.

7. Emulsion according to Claim 5 or 6, **characterized in that**, in addition to the emulsifier mixture, one or more O/W emulsifiers with an HLB value > 7 are present, where the weight ratio of emulsifier mixture to additional O/W emulsifiers is 1:9 to 9:1.

8. Emulsion according to Claim 7, **characterized in that** sodium lauroyl sarcosinate is used as additional emulsifier with an HLB value > 7.

9. Emulsion according to one of Claims 5 to 8, **characterized in that** the oil component comprises caprylic capric triglyceride.

10. Use of an emulsion according to one of Claims 5 to 9 as emulsion concentrate for producing cosmetic or pharmaceutical compositions.

## Revendications

1. Mélange d'émulsifiants, contenant
a) du stéarate de glycéryle (A)
b) du stéarate-citrate de glycéryle (B)
c) de l'alcool stéarylique (C)
d) du stéaroyllactylate de sodium (D).

2. Mélange d'émulsifiants selon la revendication 1, **caractérisé en ce que** 10 à 90% en poids des composants A et B et 90 à 10% en poids des composants C et D, par rapport à la quantité totale des composants A à D, sont présents.

3. Mélange d'émulsifiants selon la revendication 1 ou 2, **caractérisé en ce que** 30 à 80% en poids du composant A et 70 à 20% en poids du composant B, par rapport à la quantité totale des composants A et B, sont présents.

4. Mélange d'émulsifiants l'une quelconque des revendications 1 à 3, **caractérisé en ce que** 10 à 90% en poids du composant C et 90 à 10% en poids du composant D, par rapport à la quantité totale des composants C et D, sont présents.

5. Emulsion, contenant 0,5 à 20% en poids, par rapport à l'émulsion totale, d'un mélange d'émulsifiants selon l'une quelconque des revendications 1 à 4.

6. Emulsion selon la revendication 5, **caractérisée en ce qu'**il s'agit d'une émulsion H/E.

7. Emulsion selon la revendication 5 ou 6, **caractérisée en ce qu'**en plus du mélange d'émulsifiants, un ou plusieurs émulsifiants H/E présentant une valeur BHL > 7 sont présents, le rapport pondéral du mélange d'émulsifiants aux émulsifiants H/E supplémentaires étant de 1:9 à à 9:1,

8. Emulsion selon la revendication 7, **caractérisée en ce qu'**on utilise comme émulsifiant supplémentaire présentant une valeur BHL > 7 du lauroylsarcosinate de sodium.

9. Emulsion l'une quelconque des revendications 5 à 8, **caractérisée en ce que** le composant huileux contient des triglycérides capryliques/capriques.

10. Utilisation d'une émulsion selon l'une quelconque des revendications 5 à 9 comme concentrat d'émulsion destiné à la préparation de compositions cosmétiques ou pharmaceutiques.
